# EUROPÄISCHE PATENTSCHRIFT

(11) **EP 2 682 450 B1**
(45) Veröffentlichungstag und Bekanntmachung des Hinweises auf die Patenterteilung: **08.08.2018**
(21) Anmeldenummer: 13003153.7
(22) Anmeldetag: 20.06.2013
(51) Int. Cl.: C10L 3/08, C07C 1/12

(54) **VERFAHREN ZUM KATALYTISCHEN METHANISIEREN UND METHANISIERUNGSANLAGE**
METHOD FOR CATALYTIC METHANISATION AND METHANISATION SYSTEM
PROCÉDÉ DE MÉTHANISATION CATALYTIQUE ET INSTALLATION DE MÉTHANISATION

(30) Priorität: 03.07.2012 DE 102012013258
(43) Veröffentlichungstag der Anmeldung: 08.01.2014
(73) Patentinhaber: Hitachi Zosen Inova Etogas GmbH, 70565 Stuttgart (DE)
(72) Erfinder: Waldstein, Gregor, 70565 Stuttgart (DE)
(74) Vertreter: Leinweber & Zimmermann

(56) Entgegenhaltungen:
- EP-A1- 2 540 388
- DE-A1-102009 018 126
- DE-U1-202011 005 536
- US-A1- 2011 041 740

## Beschreibung

Die Erfindung betrifft ein Verfahren zum katalytischen Methanisieren eines aus einer Wasserstoffquelle bezogenen Wasserstoff und aus einer Kohlendioxidquelle bezogenes Kohlendioxid enthaltenden Eduktgases zu einem methanreichen Produktgas, bei dem die Wasserstoffquelle in einer ersten Betriebsart aus einer bereitgestellten elektrischen Energie Strom zur Wasserstofferzeugung bezieht und bei dem der Kohlendioxidquelle zur Bereitstellung des Kohlendioxids dienende thermische Energie zugeführt wird.

Derartige Methanisierungsverfahren sind bekannt und beispielsweise in der WO2011/076315 beschrieben. Die bei der Methanisierung stattfindenden chemischen Rekationen sind neben den grundsätzlich dazu ausgelegten Methanisierungsreaktoren bekannt und werden an dieser Stelle nicht weiter beschrieben. Vielmehr wird diesbezüglich auf den oben genannten Stand der Technik Bezug genommen.

Als Kohlendioxidquelle für das Eduktgas kann beispielsweise eine Biogasanlage dienen, die entweder Kohlendioxid enthaltendes Rohbiogas an das Eduktgas abgeben kann, aber auch ein aus dem Rohbiogas ausgefiltertes Kohlendioxid. Bekannterweise benötigen Biogasanlagen thermische Energie, beispielsweise für die Hygienisierung der angelieferten Biogasmasse, aber auch die Fermentierung sowie ggf. in einer Biogasaufbereitung des Rohbiogases. Da das von der Biogasanlage erzeugte Rohbiogas oder nach Aufbereitung Biomethan über seine Verbrennung eine unerschöpfliche Quelle thermischer Energie darstellt, kann die von der Biogasanlage benötigte thermische Energie besonders einfach direkt vor Ort bereitgestellt werden.

Zum anderen ist es aus der obigen Druckschrift bekannt, derartige Methanisierungsverfahren intermittierend zu betreiben. Eine nur zeitlich schwankend bereitgestellte elektrische Energie, welche z.B. aus einer nicht mit zeitlich gleichmäßiger Leistung arbeitenden erneuerbaren Energiequelle wie Wind- oder Sonnenenergie stammt, wird zur Wasserstofferzeugung genutzt. Sofern diese elektrische Energie nicht zur Verfügung steht, kann die Methanisierung gestoppt werden und der entsprechende Reaktor im Standby-Betrieb gefahren werden. Bei solchen intermittierenden Verfahren hängt die Menge des erhaltenen methanreichen Produktgases, welches beispielsweise in ein bestehendes Erdgasnetz eingespeist werden kann, somit unmittelbar von der Menge der bereitgestellten elektrischen Energie ab.

DE 10 2009 018 126 A1 offenbart ein Verfahren nach dem Oberbegriff von Anspruch 1. Im Falle einer Biogasanlage als Kohlendioxidquelle ist ein Wärmetransportpfad vorgesehen, über den Abwärme aus der Methanisierungseinrichtung zur Biogasanlage zwecks dortiger Nutzung transportiert werden kann.

DE 20 2011 005 536 U1 offenbart eine Anlage zur Nutzung von zeitlich variabel anfallendem Kohlendioxid aus verschiedenen Emissionsquellen zur ökologischen Energiegewinnung, bei der eine Methanisierungsvorrichtung vorgesehen ist und eine Vorrichtung zur Weiterverwendung des Methans, die auch die Erzeugung von Wärmeenergie erlaubt. Bei der Kohlendioxidgewinnung verwendete Reinigungs-, Separierungs- oder Verdichtungsvorrichtungen haben eine Elektroenergieversorgung, für die eine von einer Photovoltaik-Vorrichtung erzeugte Elektroenergie verwendet werden kann.

Der Erfindung liegt die Aufgabe zugrunde, ein derartiges Methanisierungsverfahren insbesondere hinsichtlich seiner Eingliederung in die Energielandschaft zu verbessern.

In verfahrenstechnischer Hinsicht wird diese Aufgabe durch eine Weiterbildung des eingangs genannten Verfahrens gelöst, die im wesentlichen dadurch gekennzeichnet ist, dass in der ersten Betriebsart die zugeführte thermische Energie wenigstens teilweise durch Umwandlung eines Teils der bereitgestellten elektrischen Energie erzeugt wird.

Dabei beruht die Erfindung auf der überraschenden Erkenntnis, dass trotz einer Umwandlung von Energie aus ihrer höchstwertigen Form, der elektrischen Energie, in eine niederwertige Form, nämlich Wärme, eine gesamtenergetisch günstige Konstellation erreicht werden kann. Auf diese Weise kann nämlich ein aus der Biogasanlage stammendes und anderweitig verbranntes Gas eingespart werden, welches entweder bereits im wesentlichen gleichwertig zu dem durch die katalytische Methanisierung hergestellten methanreichen Produktgases ist, oder jedenfalls Bestandteile enthält, welche einen Ausgangsstoff für das methanreiche Produktgas bilden. Letzteres soll einen Methangehalt von wenigstens 84%, bevorzugt von wenigstens 90% aufweisen.

Besonders zweckmäßig wird ein Teil der zugeführten thermischen Energie bereits durch von der bei der katalytischen Methanisierung und/oder der Wasserstofferzeugung erzeugten Wärme erbracht. Dieser Teil kann in der ersten Betriebsart sogar den Großteil der thermischen Energie bilden.

Die zugeführte thermische Energie kann unterschiedlichen Abnehmern auf unterschiedlichen Temperaturniveaus zugeführt werden. Beispielsweise wird eine im Rahmen der der Kohlendioxidquelle zugeordneten Biogasaufbereitung eingesetzten Aminwäsche bei der auf thermischer Basis erfolgenden Regeneration der aminhaltigen Waschflüssigkeit eingesetzte thermische Energie auf einem höheren Temperaturniveau bereitzustellen sein, als z.B. für die Hygienisierung der Biomasse.

In einer besonders bevorzugten Ausführungsform wird ein derartig erbrachter Teil der thermischen Energie, welcher aus der Wasserstofferzeugung oder der exothermen Methanisierung auf einem ersten Temperaturniveau erbracht oder bereitgestellt wird, mittels der elektrischen Energie auf ein höher gelegenes zweites Temperaturniveau gehoben. Auf diese Weise kann selbst bei einer leistungsmäßig bereits ausreichenden thermischen Energie deren Einsatzfähigkeit erhöht werden.

In einer weiteren möglichen Ausführungsform wird ein nochmals weiterer erbrachter Teil der zugeführten thermischen Energie zunächst auf einem dritten Temperaturniveau bereitgestellt und mit der elektrischen Energie auf ein höher als das dritte Temperaturniveau gelegenes viertes Temperaturniveau gehoben, welches insbesondere mit dem ersten Temperaturniveau übereinstimmt. Auf diese Weise kann wie in einer Kaskade eine Aufwertung der Temperaturniveaus erfolgen.

In einer bevorzugten Ausführungsform wird in einer zweiten Betriebsart die elektrische Energie nicht bereitgestellt. Dies eignet sich besonders für den eingangs erläuterten Fall, bei dem die Methanisierung intermittierend nur bei Verfügbarkeit aus regenerativen Energiequellen stammender elektrischer Energie durchgeführt wird. In diesem Zusammenhang kann ein Wechsel zwischen den beiden Betriebsarten im zeitlichen Mittel wenigstens einmal pro Woche, insbesondere wenigstens einmal pro Tag erfolgen.

Die Kohlendioxidquelle kann eine Biogasanlage aufweisen, auch erweitert durch eine Biogasaufbereitung in Form einer Kohlendioxidabtrennung, z.B. einer Aminwäsche, die ebenfalls bereits eingangs weiter beschrieben wurde. Grundsätzlich eignet sich das Verfahren jedoch auch für eine Kohlendioxidquelle in Form einer Abtrenneinrichtung, welche Kohlendioxid aus einer ein Kohlendioxid aufweisendes Gas bereitstellenden Gasquelle filtert.

Als Abnehmer der zugeführten thermischen Energie kommen insbesondere die Abtrenneinrichtung und davon weiter insbesondere die Regeneration der Waschflüssigkeit der Aminwäsche in Frage, aber auch Bestandteile der Biogasanlage wie Fermenter, Nachgärer und/oder eine Hygienisierung der Biomasse. Die umgewandelte elektrische Energie kann dabei zum Nutzen einer, aber auch einer beliebigen Kombination dieser Abnehmer bereitgestellt werden.

Grundsätzlich könnte mit dem methanreichen Produktgas beliebig verfahren werden, Verbrauch vor Ort, Abfüllung, etc. Besonders bevorzugt wird es jedoch in ein bestehendes Gasnetz eingespeist, wozu die zur Einspeisung in das Gasnetz erforderliche Konditionierung noch durchzuführen wäre, etwa die Brennwertanpassung, etc. Auch ein von der die Kohlendioxidquelle mit bildenden Abtrenneinrichtung erzeugtes Biomethan kann direkt in das Gasnetz eingespeist werden.

In einer zweiten Betriebsart des Verfahrens, bei dem die aus regenerativen Energiequellen stammende elektrische Energie nicht zur Verfügung steht, kann die thermische Energie bzw. Anhebung einer vorhandenen thermischen Energie auf ein höheres Temperaturniveau durch die Verbrennung von Rohbiogas oder Biomethan sichergestellt werden. Dies kann über eine einfache Verbrennung geschehen, aber auch über die Ausnutzung thermischer Abwärme in einem Blockheizkraftwerk.

In vorrichtungstechnischer Hinsicht wird die Aufgabe gelöst durch eine Methanisierungsanlage mit einem Anschluss zum Erhalt einer bereitgestellten elektrischen Energie, einem Elektrolyseur zur Wasserstofferzeugung mittels von der bereitgestellten elektrischen Energie bezogenem Strom und einem auf katalytischer Basis wirkenden Reaktor zur Erzeugung eines methanreichen Produktgases aus einem den erzeugten Wasserstoff und aus einer Kohlendioxidquelle bezogenes Kohlendioxid enthaltenden Eduktgas, und mit einer Zuführeinrichtung, mit der wenigstens ein Teil der bereitgestellten elektrischen Energie der Kohlendioxidquelle in einer in thermische Energie umgewandelten Form zuführbar ist.

Die Vorteile der erfindungsgemäßen Anlage ergeben sich aus den oben beschriebenen Vorteilen des erfindungsgemäßen Verfahrens. So ist auch insbesondere eine Steuervorrichtung vorgesehen, welche zur Steuerung der Anlage nach einem der obigen Verfahrensaspekte ausgelegt ist.

Die Zuführeinrichtung selbst kann eine Abzweigung elektrischer Energie aufweisen, welche einen nicht der Wasserstoffquelle zur Wasserstofferzeugung zugeführten Anteil der bereitgestellten elektrischen Energie zu einer Umwandlungseinrichtung abzweigt, welche die elektrische Energie in thermische Energie umwandelt, insbesondere in Form einer Erhöhung eines bereits bestehenden Temperaturniveaus.

Desweiteren wird mit der Erfindung auch ein Anlagenkomplex mit einer an eine Biogasaufbereitung enthaltende Biogasanlage angekoppelte Methanisierungsanlage wie oben angegeben unter Schutz gestellt, deren Kohlendioxidquelle von der Biogasanlage gebildet ist.

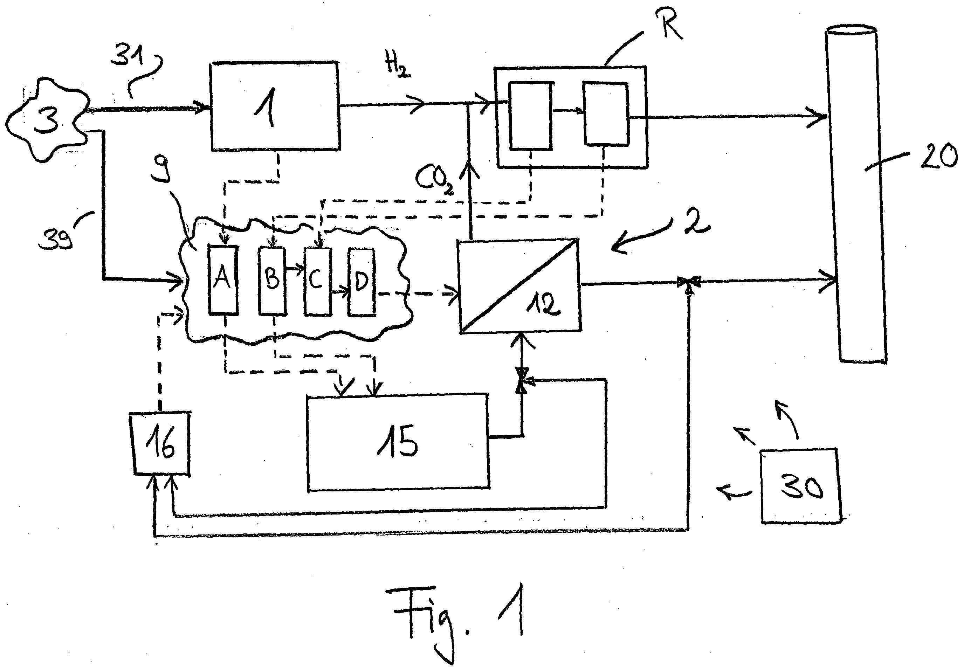

Weitere Merkmale, Einzelheiten und Vorteile der Erfindung ergeben sich aus der folgenden Beschreibung der beiliegenden Figuren, von denen
- Fig.1: das erfindungsgemäße Verfahren in einer schematischen Darstellung wiedergibt.

Gemäß der schematischen Übersicht von Fig. 1 über das Methanisierungsverfahren wird in dem in dieser Ausführungsform zweistufig ausgeführten Reaktor R ein im wesentlichen aus Wasserstoff und im zur Methanisierung richtigen stöchiometrischen Verhältnis gemischten Kohlendioxid bestehendes Eduktgas durch katalytische Methanisierung zu einem Produktgas methanisiert, welches zur Einspeisung in ein Gasnetz 20 bestimmt ist.

Das Eduktgas weist Wasserstoff H₂ auf, der aus einer von einem Elektrolyseur 1 gebildeten Wasserstoffquelle stammt. Das Kohlendioxid wird dem Reaktor R bei dieser Ausführungsform aus einer CO₂-Quelle 2 zugeführt, die durch eine Aminwäsche 12 gebildet ist. Bei der Aminwäsche 12 wird aus einem von einer Biogasanlage 15 erzeugten Rohbiogas das darin enthaltene CO₂ fast vollständig abgetrennt. Während das so aufbereitete Rohbiogas als Biomethan ebenfalls dem Gasnetz 20 zugeführt werden kann (nach entsprechender Konditionierung entsprechend den für das Gasnetz 20 geltenden Anforderungen), wird das bei der thermischen Regeneration der aminhaltigen Waschflüssigkeit wieder freigesetzte Kohlendioxid zum Reaktor R geleitet.

Die zur thermischen Regeneration der Aminwäsche zugeführte thermische Energie stammt aus einem in Fig. 1 schematisch dargestellten Wärmereservoir 9, genauer dessen Unterreservoir D. Das Temperaturniveau des Unterreservoirs D liegt in dem Bereich von 160°C bis 200°C. In diesem Ausführungsbeispiel beträgt das Temperaturniveau 170°C.

Über den Anschluss 3 wird eine elektrische Energie E_{EL} bereitgestellt, mit der die als Elektrolyseur ausgebildete H₂-Quelle 1 beschickt wird, welche elektrolytisch Wasserstoff erzeugt und dem Reaktor R zuleitet.

Die Bereitstellung der elektrischen Energie E_{EL} erfolgt hier allerdings nicht zeitlich konstant. Vielmehr stammt die bereitgestellte Energie E_{EL}, obschon sie aus dem Stromnetz bezogen wird, aus einer regenerativen Energiequelle, in diesem Ausführungsbeispiel Windenergie. Ist diese verfügbar, so steht die elektrische Energie E_{EL} zur Wasserstofferzeugung bereit und wird von dem Elektrolyseur 1 über die Leitung 31 bezogen (erste Betriebsart). Steht diese Energie nicht zur Verfügung, so wird der Elektrolyseur 1 und der Reaktor R im Standby betrieben (zweite Betriebsart).

In der ersten Betriebsart wird die bereitgestellte elektrische Energie E_{EL} allerdings nicht nur dem Elektrolyseur 1 zugeführt, sondern ein Teil davon wird über die Leitung 39 auch verwendet, um in thermische Energie umgewandelt zu werden. Dies ist schematisch mit Bezug zu dem Wärmereservoir 9 wie folgt dargestellt.

Das Wärmereservoir 9 weist in dieser Ausführungsform 4 Unterreservoirs auf, die mit A, B, C und D bezeichnet sind. In den Unterreservoirs A, B, C, D wird Wärme auf jeweils unterschiedlichen Temperaturniveaus gehalten. Das Subreservoir A beispielsweise liegt auf einem Temperaturniveau im Bereich von 65° bis 80°, in dieser Ausführungsform bei 70°. Es wird für die Wärmezufuhr bei der Hygienisierung der der Biogasanlage 15 zugeführten Biomasse eingesetzt, kann aber auch noch für ein Wärmezufuhr zu Fermentern und Nachgärern der Biogasanlage 15 herangezogen werden. Das Subreservoir A wird gespeist von der nutzbaren Abwärme des Elektrolyseurs 1.

Ein zweites Subreservoir B wird auf einem Temperaturniveau im Bereich von 80° bis 110° betrieben, in dem gezeigten Ausführungsbeispiel auf 95°C. Es wird gespeist durch die Abwärme der exothermen Methanisierungsreaktion in der zweiten Stufe des Reaktors R. Wärme aus dem Subreservoir B kann einmal ebenfalls der Biogasanlage zugeführt werden, wie in der Zeichnung schematisch angedeutet. Ein Teil der darin gespeicherten Wärmeleistung kann jedoch auch im Rahmen des Wärmereservoirs 9 auf ein höheres Niveau C angehoben werden, welches im Bereich von 110°C bis 160°C liegt, in dieser Ausführungsform bei 120°C.

Auf dem Temperaturniveau des Subreservoirs C wird aufgrund der exothermen Methanisierung gewonnene nutzbare Wärme aus der ersten Stufe des Reaktors R erzeugt, und das Reservoir damit gespeist. Das Subreservoir C kann für jegliche Zwecke in der Biogasanlage genutzt werden, für die ein darunterliegendes Temperaturniveau ausreichend ist. Jedenfalls ein Teil der auf dem Temperaturniveau des Subreservoirs C bereitgestellten thermischen Leistung, in der gezeigten Ausführungsform sogar der gesamte Teil, wird auf ein höheres Temperaturniveau gehoben, das in der Zeichnung schematisch angedeutete und oben bereits erläuterten Subreservoir D, über welches die thermische Leistung für die thermische Regeneration der aminhaltigen Waschflüssigkeit der Aminwäsche 12 bereitgestellt wird.

Dabei wird in der ersten Betriebsart die Anhebung vom Temperaturniveau des Subreservoirs C auf das Temperaturniveau des Subreservoirs D erforderliche Zusatzenergie elektrisch geleistet, und zwar bezogen von der bereitgestellten Energie E_{EL} über Leitung 39 vom Anschluss 3.

Statt der hier dargestellten vier Temperaturniveaus können auch nur drei oder zwei Temperaturniveaus gebildet werden, aber auch eine nochmals feinere Unterteilung erfolgen.

Eine in Fig. 1 nur schematisch eingezeichnete Steuereinrichtung 30 steuert in Abhängigkeit der auf den unterschiedlichen Temperaturniveaus benötigten thermischen Leistung die Verteilung des zur Umwandlung in thermische Energie herangezogenen Anteils der bereitgestellten elektrischen Energie E_{EL} auf die einzelnen Subreservoirs. Dadurch kann kaskadenartig das von der Anlage angeforderte Lastprofil an thermischer Leistung aufbauend auf dem von der Methanisierungsseite bereitgestellten Profil der nutzbaren Abwärmeleistungen erzeugt werden.

Die Art und Weise der tatsächlichen räumlichen Konstruktion der Wärmeführung, Wärmeleitungen und/oder Wärmereservoirs soll durch die rein schematische Darstellung von Fig. 1 nicht weiter eingeschränkt werden. Sie kann insbesondere getrennt oder weitergehend verzweigt sein, in Form von einzelnen getrennten Wärmekreisläufen. Auch ist der Ausdruck "Reservoir" hier nicht derartig zu verstehen, dass eine Menge an thermischer Energie gespeichert sein muss. Vielmehr kann auch vorgesehen werden, dass entsprechende Wärmekreisläufe an die Lastraten angepasste Erzeugungsraten und vice versa aufweisen, so dass teilweise oder vollständig ohne körperliche Wärmespeicher gearbeitet wird.

In der zweiten Betriebsart, in welcher die elektrische Energie E_{EL} am Anschluss 3 nicht zur Verfügung steht, bedarf es für den Fall der kontinuierlich betriebenen Biogasanlage 15 einer weiteren Quelle thermischer Energie, da die in der ersten Betriebsart eingesetzte Quelle (nutzbare Abwärme aus Elektrolyseur 1 und Reaktor R sowie elektrische Zusatzenergie E_{EL}) nicht mehr zur Verfügung steht. Dieser Bedarf der Biogasanlage 15 und der Aminwäsche 12 können dann über eine Verbrennungseinheit 16 bereitgestellt werden, der Rohbiogas und/oder Biomethan als Verbrennungsgas zugeführt wird. Die Verbrennungseinheit 16 kann dabei als einfacher Gaskessel gestaltet sein. Es kann jedoch auch ein Blockheizkraftwerk zum Einsatz kommen, dessen nicht in elektrische Energie umwandelbare Verbrennungswärme dem Reservoir 9 zugeführt wird.

Die Verbrennungseinheit 16 kann zusätzlich auch in der ersten Betriebsart einen Beitrag zur bereitgestellten thermischen Energie leisten. Gesteuert von der Steuereinrichtung 30 wird in der ersten Betriebsart jedoch wenigstens ein Teil des anderweitig zur Verbrennungsanlage 16 hin abgezweigten Rohbiogas und/oder Biomethans eingespart, da ein Teil der erforderlichen thermischen Energie über den abgezweigten Teil der bereitgestellten elektrischen Energie E_{EI} durch Umwandlung bereitgestellt wird.

Die Erfindung ist nicht auf das in der Figur 1 schematisch dargestellte Ausführungsbeispiel eingeschränkt. Vielmehr können die in den anhängigen Ansprüchen und der Beschreibung offenbarten Merkmale einzeln oder in Kombination für die Verwirklichung der Erfindung in ihren verschiedenen Ausführungsformen wesentlich sein.

## Patentansprüche

1. Verfahren zum katalytischen Methanisieren eines aus einer Wasserstoffquelle bezogenen Wasserstoff und aus einer Kohlendioxidquelle bezogenes Kohlendioxid enthaltenden Eduktgases zu einem methanreichen Produktgas, bei dem die Wasserstoffquelle in einer ersten Betriebsart aus einer bereitgestellten elektrischen Energie Strom zur Wasserstofferzeugung bezieht und bei dem der Kohlendioxidquelle zur Bereitstellung des Kohlendioxids dienende thermische Energie zugeführt wird,
**dadurch gekennzeichnet, dass**
in der ersten Betriebsart die zugeführte thermische Energie wenigstens teilweise durch Umwandlung eines Teils der bereitgestellten elektrischen Energie erzeugt wird.

2. Verfahren nach Anspruch 1, bei dem ein weiterer Teil der zugeführten thermischen Energie durch von der bei der katalytischen Methanisierung und/oder der Wasserstofferzeugung erzeugten Wärme erbracht wird.

3. Verfahren nach Anspruch 1 oder 2, bei dem die zugeführte thermische Energie unterschiedlichen Abnehmern auf unterschiedlichen Temperaturniveaus zugeführt wird.

4. Verfahren nach einem der vorhergehenden Ansprüche, bei dem ein/der weitere erbrachte Teil der zugeführten thermischen Energie zunächst auf einem ersten Temperaturniveau bereitgestellt wird und mit der elektrischen Energie auf ein höher als das erste Temperaturniveau gelegenes zweites Temperaturniveau gebracht wird.

5. Verfahren nach Anspruch 4, bei dem ein nochmals weiterer erbrachter Teil der zugeführten thermischen Energie zunächst auf einem dritten Temperaturniveau bereitgestellt wird und mit der elektrischen Energie auf ein höher als das dritte Temperaturniveau gelegenes viertes Temperaturniveau gebracht wird, welches insbesondere mit dem ersten Temperaturniveau übereinstimmt.

6. Verfahren nach einem der vorhergehenden Ansprüche, bei dem in einer zweiten Betriebsart die elektrische Energie nicht bereitgestellt ist.

7. Verfahren nach Anspruch 6, bei der ein Wechsel zwischen den beiden Betriebsarten durchschnittlich wenigstens einmal pro Woche, insbesondere wenigstens einmal pro Tag erfolgt.

8. Verfahren nach einem der vorhergehenden Ansprüche, bei dem die Kohlendioxidquelle eine an eine Gasquelle, insbesondere Rohbiogasquelle angeschlossene Abtrenneinrichtung zur Abtrennung von Kohlendioxid aus dem von der Gasquelle bereitgestellten Gas aufweist, insbesondere eine Aminwäsche.

9. Verfahren nach einem der vorhergehenden Ansprüche, bei dem die Kohlendioxidquelle eine Biogasanlage aufweist.

10. Verfahren nach einem der Ansprüche 8 oder 9, bei dem die Abnehmer der zugeführten thermischen Energie aus der Gruppe Abtrenneinrichtung, insbesondere Regeneration der Waschflüssigkeit der Aminwäsche, Fermenter, Nachgärer und/oder Hygienisierung der Biogasanlage ausgewählt ist.

11. Verfahren nach einem der vorhergehenden Ansprüche, bei dem das methanreiche Produktgas und ggf. auch ein von der an die Rohbiogasquelle angeschlossene Abtrenneinrichtung erzeugtes Biomethan in ein bestehendes Gasnetz eingespeist wird.

12. Verfahren nach einem der Ansprüche 6 bis 11, bei dem die Zuführung der thermischen Energie in der zweiten Betriebsart durch Verbrennung eines Brenngases, insbesondere dem von der Biogasanlage nebst Biogasaufbereitungsanlage erzeugten Rohbiogas und/oder Biomethan gestützt wird.

13. Methanisierungsanlage, mit
einem Anschluss (3) zum Erhalt einer bereitgestellten elektrischen Energie (E_{EL}).
einem Elektrolyseur (1) zur Wasserstofferzeugung mittels von der bereitgestellten elektrischen Energie (Eₑₗ) bezogenem Strom und
einem auf katalytischer Basis wirkenden Reaktor (R) zur Erzeugung eines methanreichen Produktgases aus einem den erzeugten Wasserstoff und aus einer Kohlendioxidquelle (2) bezogenes Kohlendioxid enthaltenden Eduktgas,
**gekennzeichnet durch** eine Zuführeinrichtung (39, 9), mit der wenigstens ein Teil der bereitgestellten elektrischen Energie der Kohlendioxidquelle in einer in thermische Energie umgewandelten Form zuführbar ist.

14. Methanisierungsanlage nach Anspruch 13, mit einer Steuervorrichtung (30), welche zur Steuerung der Anlage nach einem der Ansprüche 1 bis 12 ausgelegt ist.

15. Anlagenkomplex mit einer an eine eine Biogasaufbereitung (12) enthaltende Biogasanlage (15) angekoppelte Methanisierungsanlage nach Anspruch 13 oder 14, deren Kohlendioxidquelle von der Biogasanlage gebildet ist.

## Claims

1. A method for the catalytic methanisation of a hydrogen obtained from a hydrogen source and of educt gas containing carbon dioxide obtained from a carbon dioxide source to form a methane-rich product gas, wherein the hydrogen source in a first operating mode draws current from electrical energy that is provided to generate hydrogen, and wherein thermal energy serving to provide the carbon dioxide is supplied to the carbon dioxide source,
**characterised in that**
in the first operating mode the thermal energy that is supplied is generated at least partially by converting a part of the electrical energy that is provided.

2. The method according to Claim 1, wherein a further part of the thermal energy that is supplied is provided by heat generated by the catalytic methanisation and/or the generation of hydrogen.

3. The method according to Claim 1 or 2, wherein the thermal energy that is supplied is supplied to different consumers at different temperature levels.

4. The method according to any of the preceding claims, wherein a/the further part of the thermal energy that is supplied is first of all provided at a first temperature level and is brought with the electrical energy to a second temperature level that is higher than the first temperature level.

5. The method according to Claim 4, wherein yet a further part of the thermal energy that is supplied is first of all provided at a third temperature level and is brought with the electrical energy to a fourth temperature level which is higher than the third temperature level and which in particular corresponds to the first temperature level.

6. The method according to any of the preceding claims, wherein the electrical energy is not provided in a second operating mode.

7. The method according to Claim 6, wherein a change between the two operating modes takes place on average at least once a week, in particular at least once a day.

8. The method according to any of the preceding claims, wherein the carbon dioxide source has a separating device connected to a gas source, in particular a raw biogas source, for separating carbon dioxide from the gas provided by the gas source, in particular an amine scrubber.

9. The method according to any of the preceding claims, wherein the carbon dioxide source comprises a biogas plant.

10. The method according to either of Claims 8 or 9, wherein the consumers of the thermal energy that is supplied are selected from the group separating device, in particular regeneration of the washing liquid of the amine scrubber, fermenter, secondary fermenter and/or sanitisation of the biogas plant.

11. The method according to any of the preceding claims, wherein the methane-rich product gas and optionally also a biomethane generated by the separating device connected to the raw biogas source is fed into an existing gas network.

12. The method according to any of Claims 6 to 11, wherein the supply of the thermal energy in the second operating mode is supported by the combustion of a fuel gas, in particular the raw biogas and/or biomethane generated by the biogas plant together with the biogas processing plant.

13. A methanisation system comprising
a connection (3) for receiving electrical energy that is provided (E_{EL}),
an electrolyzer (1) for generating hydrogen by means of current obtained from the electrical energy (Eₑₗ) that is provided, and
a reactor (R) operating on a catalytic basis for generating a methane-rich product gas from an educt gas containing the hydrogen that is generated and carbon dioxide obtained from a carbon dioxide source (2).
**characterised by** a supply device (39, 9) with which at least a part of the electrical energy that is provided can be supplied to the carbon dioxide source in a form converted into thermal energy.

14. The methanisation system according to Claim 13, comprising a control device (30) which is designed to control the system according to any of Claims 1 to 12.

15. A plant complex comprising a methanisation system coupled to a biogas plant (15) containing a biogas processor (12) according to Claim 13 or 14, the carbon dioxide source of which is formed by the biogas plant.

## Revendications

1. Procédé de méthanisation catalytique d'un éduit gazeux contenant de l'hydrogène provenant d'une source d'hydrogène et du dioxyde de carbone provenant d'une source de dioxyde de carbone afin de produire un produit gazeux riche en méthane, dans lequel la source d'hydrogène, dans un premier mode d'exploitation, consomme du courant issu d'une énergie électrique fournie en vue de la production d'hydrogène et dans lequel de l'énergie thermique servant à fournir le dioxyde de carbone est apportée à la source de dioxyde de carbone,
**caractérisé en ce que**
dans le premier mode d'exploitation, l'énergie thermique apportée est produite au moins partiellement par transformation d'une partie de l'énergie électrique fournie.

2. Procédé selon la revendication 1, dans lequel une autre partie de l'énergie thermique apportée est dérivée de la chaleur produite lors de la méthanisation catalytique et/ou de la production d'hydrogène.

3. Procédé selon la revendication 1 ou 2, dans lequel l'énergie thermique apportée est apportée à différents consommateurs à différents niveaux de température.

4. Procédé selon l'une des revendications précédentes, dans lequel une/l'autre partie, dérivée, de l'énergie thermique apportée est tout d'abord fournie à un premier niveau de température puis est portée à un deuxième niveau de température supérieur au premier niveau de température à l'aide de l'énergie électrique.

5. Procédé selon la revendication 4, dans lequel encore une autre partie, dérivée, de l'énergie thermique apportée est tout d'abord fournie à un troisième niveau de température puis est portée à un quatrième niveau de température supérieur au troisième niveau de température à l'aide de l'énergie électrique, lequel quatrième niveau de température correspond notamment au premier niveau de température.

6. Procédé selon l'une des revendications précédentes, dans lequel, dans un deuxième mode d'exploitation, il n'est fourni aucune énergie électrique.

7. Procédé selon la revendication 6, dans lequel un changement entre les deux modes d'exploitation est réalisé en moyenne au moins une fois par semaine, notamment au moins une fois par jour.

8. Procédé selon l'une des revendications précédentes, dans lequel la source de dioxyde de carbone présente un dispositif de séparation, notamment une unité de lavage aux aminés, visant à séparer le dioxyde de carbone du gaz fourni par une source de gaz à laquelle est relié ledit dispositif de séparation, notamment une source de biogaz brut.

9. Procédé selon l'une des revendications précédentes, dans lequel la source de dioxyde de carbone présente une installation de biogaz.

10. Procédé selon l'une des revendications 8 et 9, dans lequel les consommateurs d'énergie thermique apportée sont choisis dans le groupe constitué par un dispositif de séparation, notamment une unité de régénération du liquide lavage de l'unité de lavage aux aminés, un fermenteur, un post-digesteur et/ou une unité d'hygiénisation de l'installation de biogaz.

11. Procédé selon l'une des revendications précédentes, dans lequel le produit gazeux riche en méthane, et éventuellement également du biométhane produit par le dispositif de séparation relié à la source de biogaz brut, est injecté dans un réseau de gaz existant.

12. Procédé selon l'une des revendications 6 à 11, dans lequel l'apport en énergie thermique dans le deuxième mode d'exploitation est appuyé par la combustion d'un gaz combustible, notamment du biogaz brut et/ou du biométhane produit par l'installation de biogaz en sus d'une unité de conditionnement du biogaz.

13. Installation de méthanisation, comportant :
une connexion (3) permettant d'obtenir la fourniture d'énergie électrique (E_{EL}),
un électrolyseur (1) destiné à produire de l'hydrogène au moyen de l'électricité provenant de l'énergie électrique (E_{EL}) fournie, et
un réacteur (R) à action catalytique destiné à produire un produit gazeux riche en méthane à partir d'un éduit gazeux contenant l'hydrogène produit et du dioxyde de carbone provenant d'une source de dioxyde de carbone (2),
**caractérisée par** un dispositif d'amenée (39, 9) au moyen duquel au moins une partie de l'énergie électrique fournie est apportée à la source de dioxyde de carbone sous une forme transformée en énergie thermique.

14. Installation de méthanisation selon la revendication 13, comportant un dispositif de commande (30) conçue pour commander l'installation selon l'une des revendications 1 à 12.

15. Complexe d'installations comportant une installation de méthanisation selon la revendication 13 ou 14 couplée à une installation de biogaz (15) contenant une unité de conditionnement de biogaz (12), la source de dioxyde de carbone de l'installation de méthanisation étant constituée par l'installation de biogaz.
